(19) Europäisches Patentamt
European Patent Office
Office européen des brevets

(11) Veröffentlichungsnummer: **0 257 418 B1**

(12) # EUROPÄISCHE PATENTSCHRIFT

(45) Veröffentlichungstag der Patentschrift: **19.05.93**

(51) Int. Cl.5: **C07H 17/04**, A61K 31/70

(21) Anmeldenummer: **87111510.1**

(22) Anmeldetag: **08.08.87**

(54) **Oxiran-Pseudooligosaccharide, Verfahren zu deren Herstellung, deren Verwendung und pharmazeutische Präparate.**

(30) Priorität: **13.08.86 DE 3627421**

(43) Veröffentlichungstag der Anmeldung:
**02.03.88 Patentblatt 88/09**

(45) Bekanntmachung des Hinweises auf die Patenterteilung:
**19.05.93 Patentblatt 93/20**

(84) Benannte Vertragsstaaten:
**AT BE CH DE ES FR GB GR IT LI LU NL SE**

(56) Entgegenhaltungen:
**EP-A- 0 089 812**
**EP-A- 0 173 948**
**EP-A- 0 173 950**

(73) Patentinhaber: **HOECHST AKTIENGESELL-SCHAFT**
**Postfach 80 03 20**
**W-6230 Frankfurt am Main 80(DE)**

(72) Erfinder: **Vértesy, Lászl , Dr.**
**Eppenhainer Weg 6**
**W-6239 Eppstein/Taunus(DE)**
Erfinder: **Betz, Joachim, Dr.**
**Hohe Stätte 16**
**W-6000 Frankfurt am Main 56(DE)**
Erfinder: **Fehlhaber, Hans-Wolfram, Dr.**
**Thomas-Mann-Strasse 5a**
**W-6270 Idstein(DE)**
Erfinder: **Geisen, Karl, Dr.**
**Jahnstrasse 43**
**W-6000 Frankfurt am Main(DE)**

**EP 0 257 418 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft biologisch aktive Oxiran-Pseudooligosaccharide und ihre physiologisch verträglichen Salze. Sie besitzen α-Glukosidase, d.h. z.B. α-Amylase- und Disaccharidase-hemmende Eigenschaften und können daher in der Human- und Tiermedizin, in der Tierernährung sowie in der Biotechnologie der Stärke verwendet werden.

In der Europäischen Patentanmeldung mit der Veröffentlichungs-Nr. 0 173 948 (EP-A2-0 173 948 entspr. US-Patent 4 632 917) werden Pseudooligosaccharide mit α-Glukosidase-hemmender Wirkung und der Formel III

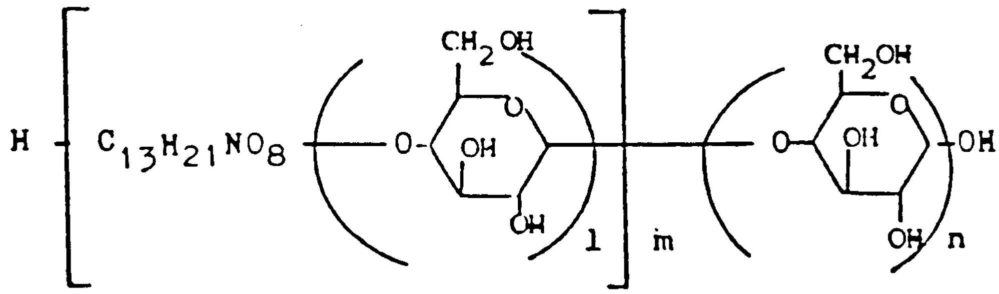

III

in welcher
l = 1 oder 2
m = 1, 2 oder 3
n = eine ganze Zahl von 1 bis 20 bedeutet,
beansprucht.

Bei den beschriebenen Verbindungen handelt es sich um potente Inhibitoren, im folgenden auch als Inhibitoren vom W-46 Typ bezeichnet, die aufgrund ihrer Wirkung auf Glukosid-Hydrolasen des Verdauungsextraktes als Resorptionsverzögerer von Glukose bei den Behandlungen von Diabetes, Adipositas u. a. eingesetzt werden können. Sie werden aus den Kulturflüssigkeiten von Inhibitor W-46 bildenden Organismen wie z.B. Streptomyces galbus subsp. FH 1716 (DSM 3007) gewonnen. Man erhält nur nach einem aufwendigen Verfahren einheitliche Substanzen, vielfach gewinnt man eine Mischung von verschiedenen Inhibitoren, die sich in der Länge der Glukose-enthaltenden Ketten unterschieden. Diese Uneinheitlichkeit ist für eine sichere Dosierung und Standardisierung hinderlich, sie ist bei der Anwendung des Präparats von Nachteil.

Es ist nun gefunden worden, daß sich durch saure Hydrolyse oder enzymatische Glukoseabspaltung aus dem nativen Inhibitorgemisch entsprechend der Anmeldung EP-A2 0 173 948 neue, hochaktive Abbauprodukte gewinnen lassen, die mit geeigneten Methoden leicht voneinander getrennt und in reiner Form hergestellt werden können.

Die Erfindung betrifft daher Oxiran-Pseudooligosaccharide der Formel I

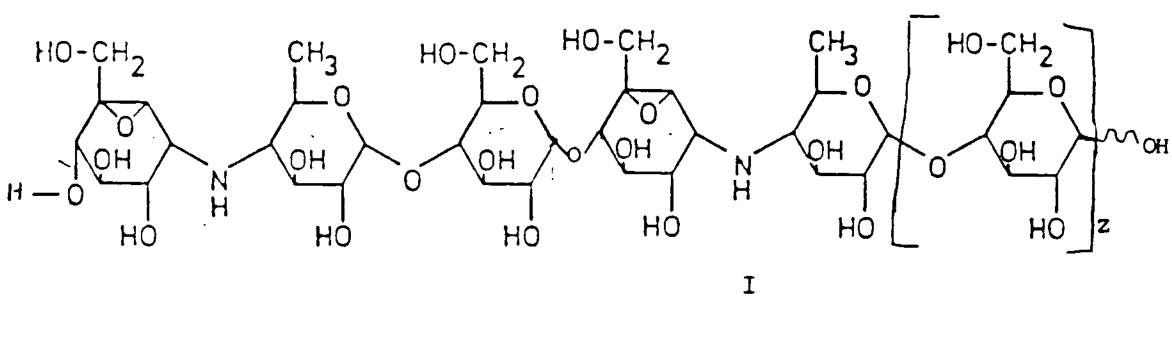

I

worin
z = null oder 1 ist,
sowie deren physiologisch verträgliche Salze mit Säuren. Die beiden unter die Formel I fallenden Inhibitoren weisen die folgenden Formeln Ia und Ib

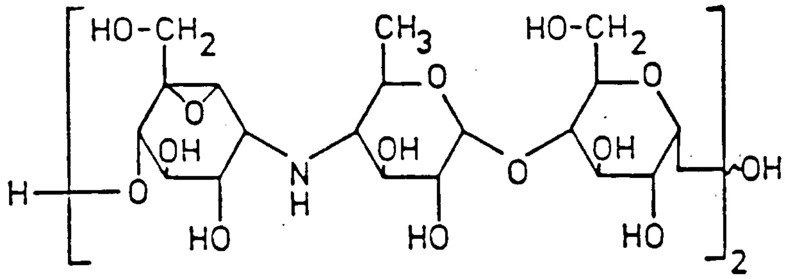

(Ia)

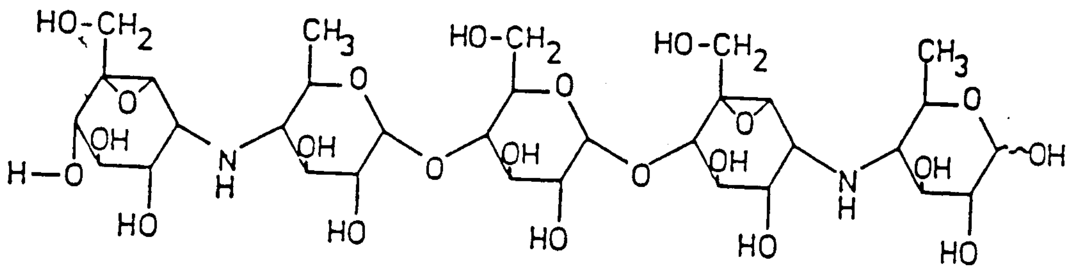

(Ib)

auf. Das Pseudooligosaccharid der Formel Ia und seine Salze werden in den folgenden Ausführungen auch als Inhibitor W-46- H und das Pseudooligosaccharid der Formel Ib sowie seine Salze als Inhibitor W-46 P bezeichnet.

Die Erfindung betrifft ferner Verfahren zur Herstellung der Inhibitoren W-46 H und P, pharmazeutische Präparate, die diese Verbindungen enthalten und ihre Verwendung als Arzneimittel, Diagnostikum und Reagenz.

Das Verfahren zur Herstellung der Inhibitoren W-46 H und W-46 P ist dadurch gekennzeichnet, daß man

a) von einem $\alpha$-Glukosidaseinhibitor der Formel II

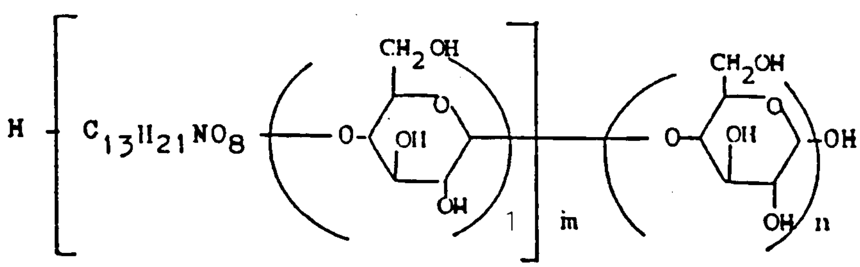

II

in welcher

m = 2 oder 3

n = eine ganze Zahl von 1 bis 20 bedeutet,

oder von einem Gemisch dieser Inhibitoren mit chemischen oder biochemischen Methoden unter Bildung einer Verbindung der Formel I Zucker abspaltet, oder

b) einen Pseudoologosaccharide der Formel I erzeugenden Streptomyceten in einem Fermentationsmedium in geeignetem Submersverfahren kultiviert, die Inhibitoren aus dem Mycel oder dem Kulturfiltrat in an sich bekannter Weise isoliert und reinigt und die erhaltenen Verbindungen der Formel I gegebenenfalls in ein physiologisch verträgliches Salz überführt.

Von den Streptomyceten eignet sich insbesondere Streptomyces galbus subsp. FH 1716 für die Durchführung des Verfahrens. Dieser Stamm ist bei der Deutschen Sammlung von Mikroorganismen (DMS) unter der Regiestrier-Nr. DSM 3007 hinterlegt und in EP-A2-0 173 948 beschrieben. Es können aber auch die Varianten und Mutanten dieses Stammes zur Gewinnung der Inhibitoren W-46 H und W-46 P eingesetzt werden.

Inhibitoren der Formel II und Verfahren zu ihrer Gewinnung sind in EP-A2-0 173 948 beschrieben. Durch Abspaltung von Zucker wie z.B. Glukose entstehen nach dem erfindungsgemäßen Verfahren an der

Saccharidkette verkürzte, einheitliche Inhibitoren.

Die Inhibitoren W-46 H und W-46 P werden vorzugsweise nach Methode a gewonnen.

Bei der Gewinnung geht man zwecksmäßigerweise wie folgt vor:

Als Ausgangsmaterial dienen Inhibitoren der Formel II (vergl. EP-A2-0 173 948) entweder in angereicherter oder in chemisch gereinigter Form. Es können aber auch die rohen, ungereinigten Fermentationslösungen von W-46 bildenden Organismen verwendet werden. Die Abspaltung der verschieden langen Glukoseketten der Inhibitoren erfolgt z.B. durch saure Hydrolyse mit Schwefelsäure, Salzsäure, Trifluoressigsäure u.a. im Temperaturbereich 0° bis 120°C, vorzugsweise 80 - 105°C. Die Hydrolysedauer beträgt in Abhängigkeit von der Temperatur einige Minuten bis mehrere Tage. Vorzugsweise arbeitet man im Bereich von 20 - 200 Minuten.

Eine andere Möglichkeit zur Abspaltung von Neutralzuckern wie z.B. Maltose und Glukose von den Inhibitoren des Gemisches gemäß EP-A2-0 173 948 besteht in der Anwendung von $\alpha$-Glukosid-spaltenden Enzymen. Es ist gefunden worden, daß im Gegensatz zu Warmblüterenzymen einige mikrobielle $\alpha$-Amylasen sehr wohl in der Lage sind, die Inhibitoren vom W-46 Typ wie z.B. W-46 A, W-46 B oder W-46 C u.a. mehr (vergl. EP-A2-0 173 948) durch Neutralzuckerabspaltung zu verkürzen und damit zu vereinheitlichen. Solche Enzyme sind z.B. die $\alpha$-Amylase aus Bacillus subtilis oder Bacillus licheniformis (beide besonders geeignet für die Gewinnung von W-46 H), es können aber auch Amylasen aus anderen geeigneten Mikroorganismen verwendet werden. Bevorzugt eingesetzt werden $\alpha$-Amylasen aus thermophilen oder thermotolerablen Mikroorganismen, deren Enzyme auch ein höheres Temperaturoptimum haben. Solche Amylasen führen bei den erhöhten Temperaturen zu höheren Reaktionsrate, d.h. zu vorteilhaften, kürzeren Reaktionszeiten. Mit Hilfe solcher geeigneter Enzyme lassen sich 0.01 bis 20 vorzugsweise 0.5 - 5 prozentiger Inhibitorlösungen in guter Ausbeute umsetzen. Der pH-Wert und die Temperatur werden hierbei in Abhängigkeit von den Enzymeigenschaften gewählt. Gearbeitet weden kann zwischen 0° und 100°C, bevorzugt wird der Bereich zwischen 30° und 80°C.

Durch Steuerung der Fermentationszeit bei der mikrobiellen Herstellung (gemäß Verfahren b) oder Variation der Glukoseabspaltung oder der Hydrolysezeit (gemäß Verfahren a) kann entweder der Inhibitor W-46 H oder W-46 P bevorzugt gewonnen werden. Aus den Fermentations- oder Reaktionslösungen können die Inhibitoren durch an sich bekannte Verfahren isoliert und gereinigt werden.

Hierzu eignet sich eine große Zahl von Verfahren, wie beispielsweise Chromatographie an Ionenaustauschern, Molekularsieben oder Adsorptionsharzen, darüberhinaus Lösungsmittelfällungen, Ultrafiltration, Craig-Verteilung u.a.

Ein bevorzugtes Verfahren zur Isolierung und Reinigung der Inhibitoren W-46 H bzw. P besteht darin, daß man die Inhibitoren aus dem - z.B. wie vorstehend beschrieben - behandelten Kulturfiltrat oder der Reaktionslösung an ein geeignetes Harz z.B. auf Polystyrolbasis adsorbiert, dieses beladene Harz abtrennt und die genannten Inhibitoren durch Elution mit geeigneten Pufferlösungen wie z.B. Phosphat- oder Na-acetat-Pufferlösung oder mit ggfs. wasserhaltigen organischen Lösungsmitteln, wie z.B. Methanol, Ethanol, Aceton, vorzugsweise aber mit wäßrigem Isopropanol, isoliert. Die inhibitorhaltigen Eluate konzentriert man durch Ultrafiltration in an sich bekannter Weise, wobei gleichzeitig eine Entsalzung vorgenommen wird. Die ionenarme wäßrige Lösung der erwähnten Inhibitoren wird dann auf einer Ionenaustauscher-Säule chromatographisch in an sich bekannter Weise aufgetrennt. Vorzugsweise verwendet man stark oder schwach saure Kationenaustauscher z.B. auf Styrol-Divinylbenzol-Copolymerisatbasis, welche als funktionelle Gruppen $SO_3H$ oder -COOH Gruppen tragen ($^R$Dowex 50 W, $^R$Amberlite CG 120) oder auf Basis modifizierter Sulfopropyl-Cellulose (SP-$^R$Sephadex) als Ionenaustauscher, brauchbar ist aber auch eine große Anzahl anderer käuflicher Kationenaustauscher. Der letzte Schritt der Isolierung ist die Anwendung eines Molekularsiebes, z.B. auf Polyacrylamid-Gel-Basis ($^R$Biogel P-6) oder auf Basis modifizierter Cellulose ($^R$Sephadex). Die resultierenden wäßrigen Lösungen des reinen Materials werden dann z.B. durch Lyophilisierung getrocknet.

Die reinen Inhibitoren W-46 H und W-46 P sind farblose, amorphe Pseudooligosaccharide. Sie enthalten Stickstoff und haben schwach basischen Charakter. So wandern die Inhibitoren W-46 H und P in der Hochspannungselektrophorse in sauren Puffern, wie z.B. wäßrigen Ameisensäure/Essigsäure Mischungen als Kationen in Richtung Kathode. Die erfindungsgemäßigen Substanzen besitzen reduzierende Eigenschaften, die, wie in der Zuckerchemie üblich, z.B. mit Triphenyltetrazoliumchlorid (TCC) nachgewiesen werden können.

Die durch FAB-Massenspektrometrie bestimmten Molekulargewichte der reinen Verbindungen betragen: 981 m/z (M + $H^+$) entsprechend der Summenformel $C_{38}H_{64}N_2O_{27}$ des Inhibitors W-46 H (freie Base), bzw. 819 m/z (M + $H^+$) entsprechend der Summenformel $C_{32}H_{54}N_2O_{22}$ des Inhibitors W-46 P (freie Base). Durch spektroskopische, insbesondere kernresonanzspektroskopische Untersuchungen wurden den Verbindungen

W-46 H die Formel Ia und
W-46 P die Formel Ib zugeordnet.

In der Literatur sind bereits mehrere α-Glukosidaseinhibitoren beschrieben worden, jedoch nur die von H. Takeda et al. (Japan KOKAI 83-172.400 (11. Oktober 1983)) beschriebene Verbindungsreihe: NS 1 bis NS 17 enthält Epoxydringe (Oxirane) in den Strukturformeln. Die Epoxid-Strukturen kommen jeweils nur einmal vor.

Durch die Formeln Ia und Ib, in denen die wirksamen Epoxyd-psuedoaminozucker-Strukturen zweifach vorhanden sind, unterscheiden sich die erfindungsgemäßen Verbindungen von den Inhibitoren der Reihe NS 1 bis NS 17. Gegenüber den Pseudooligosacchariden mit α-Glukosidase-hemmender Wirkung gemäß EP-A2-0 173 948 sind W-46 H und W-46 P durch die verkürzten Saccharidketten (geringeres Molekulargewicht) unterscheidbar. Somit liegen hier neue Substanzen vor, die eine einheitliche Struktur aufweisen.

Sowohl der Inhibitor W-46 H als auch W-46 P hemmen die α-Amylase aus Pankreas und die Disaccharidasen aus Dünndarmmucosa, insbesondere die rohrzuckerspaltende Saccharase stark. Die spezifischen Aktivitäten betragen 4 x $10^4$ α-Amylaseinhibitoreneinheiten (AIE) per mg bzw. 1 x $10^4$ Saccharaseinhibitoreinheit (SIE) per mg für W-46 H und 1 x $10^4$ AIE per mg bzw. 3 x $10^4$ SIE per mg für den Inhibitor W-46 P. Die Aktivitäten wurden in folgenden Tests bestimmt:

AMYLASETEST

Eine Amylase-Inhibitoreinheit (AIE) ist definiert als die Inhibitormenge, die unter den Testbedingungen zwei Amylase-Einheiten (AE) zu 50 % zu hemmen vermag. Eine Amylase-Einheit ist nach internationaler Übereinkunft die Enzymmenge, die in einer Minute 1 μ Äquivalent glucosidischer Bindungen in der Stärke spaltet. Die μVal gespaltener glucosidischer Bindungen werden als μVal reduzierender Zucker photometrisch mit Dinitrosalizylsäure bestimmt. Die Angaben sind als μMole Maltose berechnet, die anhand einer Maltose-Eichgeraden ermittelt werden.

Die Tests werden folgendermaßen durchgeführt:
α-Amylase aus Schweinepankreas und die zu testenden Lösungen werden gemeinsam in 1,0 ml 20 mM Phosphatpuffer, pH 6,9 + 10mM NaCl 10 - 20 min. bei 37°C vorinkubiert. Die enzymatische Reaktion wird durch Zugabe von 1,0 ml löslicher Stärke (0,25 % in dem angegebenen Puffer) nach Zulkowski gestartet. Nach genau 10 min. wird die Reaktion mit 2,0 ml Dinitrosalizylsäure- Farbreagenz (nach Boehringer Mannheim: Biochemica-Information II) abgestoppt und zur Farbentwicklung 5 min. im siedenden Wasserbad erhitzt. Nach dem Abkühlen wird die Extinktion bei 546 nm gegen den Reagentienleerwert gemessen. Die 50%ige Hemmung wird im Vergleich zur ungehemmten Enzymreaktion durch Einsatz verschiedener Inhibitormengen graphisch mittels der Wahrscheinlichkeitsauftragung bestimmt.

SACCHARASE-TEST

Eine Saccharase-Inhibitoreinheit (SIE) ist definiert als die Inhibitormenge, die unter den Testbedingungen zwei Saccharase-Einheiten (SE) zu 50 % zu hemmen vermag. Eine SE ist nach internationaler Übereinkunft die Enzymmenge, die in einer Minute 1 μ Äquivalent glycosidischer Bindungen der Saccharose spaltet. Die μVal gespaltener glucosidischer Bindungen werden als 2 μVal photomerisch mit der Hexokinase/Glukose-6-Phosphatdehydrogenase-Methode bestimmt. Die Angaben sind als μMole Hexosen berechnet, die anhand einer Glukose Eichgeraden ermittelt werden.

Die Tests wurden nach H. U. Bergmeyer, beschrieben in "Methods of Enzymatic Analysis", 3rd edition, Verlag Chemie, Weinheim, 1984, Seite 96 - 103, durchgeführt.

Die Eigenschaften der erfindungsgemäßen Inhibitoren sind im Hinblick auf die Anwendung als Therapeutikum gegen Diabetes und Prädiabetes sowie Adipositas und Unterstützung von Diät interessant. Aufgrund ihrere Eigenschaften sind sie auch als Reagenz für diagnostische Zwecke wertvoll. Die Erfindung betrifft daher auch Arzneimittel, insbesondere solche zur Behandlung der genannten Krankheiten, sowie die Verwendung als Arzneimittel, insbesondere als Antidiabetikum, sowie als Reagenz.

Stärke- und rohrzuckerhaltige Nahrungs- und Genußmittel führen bei Tier und Mensch zu einem Anstieg des Blutzuckers und dadurch auch zu einer verhmehrten Insulinsekretion des Pankreas. Die Hyperglykämie kommt durch die Aufspaltung der Stärke und des Rohrzuckers im Verdauungstrakt unter Einwirkung von Amylase und Saccharase zu Glukose zustande.

Bei Diabetikern ist die Hyperglykämie besonders ausgeprägt und lang anhaltend.

Die alimentäre Hyperglykämie sowie die Hyperinsulinämie nach Stärke- und Rohrzuckeraufnahme läßt sich durch die erfindungsgemäßen Inhibitoren W-46 H und W-46 P vermindern. Diese Wirkung ist

dosisabhängig. Die erfindungsgemäßen α-Glukosidase-inhibitoren lassen sich daher als Therapeutikum einsetzen bei Diabetes, Prädiabetes und Adipositas sowie zur Unterstützung von Diät. Zu diesem Zweck empfiehlt sich eine Verabreicherung, insbesondere zu den Mahlzeiten. Die Dosierung, die sich nach dem Gewicht des Patienten sowie dem individuellen Bedarf ausrichten soll, beträgt 5 – 500 mg pro Dosis, die zweckmäßig zu jeder Mahlzeit genommen wird. Die Dosierung kann jedoch in begründeten Einzelfällen auch darüber oder darunter liegen.

Die erfindungsgemäßen α-Glukosidase-Inhibitoren eignen sich insbesondere zur oralen Verabreichung. Sie können als Substanz per se, als ihre physiologisch verträglichen Salze mit Säuren, aber auch in Form einer pharmazeutischen Zubereitung unter Verwendung der üblichen Hilfs- und Trägerstoffe angewandt werden. Auch eine kombinierte Anwendung mit anderen Arzneimitteln, wie blutzuckersenkenden oder lipidsenkenden Substanzen, kann von Vorteil sein. Da höhermolekulare Saccharide als solche nicht oder nicht nennenswert aus dem Verdauungstrakt resorbiert werden, sind von den erfindungsgemäßen Substanzen keine toxikologisch bedenklichen Nebenwirkungen zu erwarten.

Dementsprechend konnten nach oraler Gabe auch hoher Dosen der Inhibitoren W-46 H und P an Versuchstieren keine auffälligen Symptome erkannt werden.

Zur Prüfung der pharmakologischen Wirkung der α-Glukosidase-Inhibitoren erhielten nüchterne, männliche Wistar-Ratten mit einem Gewicht zwischen 200 und 250 g einen erfindungsgemäßen Hemmstoff W-46 H oder W-46 P zusammen mit 2 g Stärke bzw. Rohrzucker pro kg Körpergewicht als Suspension in®Tylose (Methyl-hydroxyethyl-cellulose) oral verabreicht. Durch Bestimmung von Blutzuckerkonzentrationen in Blutproben, die vor, während und nach p.o. Applikation der α-Glukosidase-Inhibitoren entnommen worden sind, wurde die Wirksamkeit der Präparate bewiesen.

In diesen Untersuchungen wurden für den Inhibitor W-46 H die in den Tabellen 1 (Wirkung auf Blutglukosekonzentration der Stärke-belasteten Ratte) und 2 (Wirkung auf die Blutglukosekonzentration der Rohrzucker-belasteten Ratten) zusammengestellte Werte ermittelt.

TABELLE 1

| Zeit in Stunden nach der Behandlung | Blutglukose in mmol/l ($\bar{x}$ ± SEM, n = 7) | | |
|---|---|---|---|
| | p.o. Dosis 0.3 mg/kg | p.o. Dosis 1.0 mg/kg | Kontrolle |
| 0 | 3.77 ± 0.09 | 3.93 ± 0.10 | 3.79 ± 0.14 |
| 0.5 | 5.53 ± 0.17 | 5.04 ± 0.08 | 5.56 ± 0.16 |
| 1 | 5.59 ± 0.06 | 5.07 ± 0.09 | 6.36 ± 0.17 |
| 2 | 5.67 ± 0.13 | 4.99 ± 0.14 | 5.18 ± 0.17 |
| 3 | 4.86 ± 0.18 | 4.57 ± 0.10 | 4.94 ± 0.16 |
| 5 | 4.39 ± 0.13 | 4.13 ± 0.08 | 4.19 ± 0.14 |

TABELLE 2

| Zeit in Minuten nach der Behandlung | Blutglukose in mmol/l ($\bar{x}$ ± SEM, n = 7) | | | |
|---|---|---|---|---|
| | p.o. Dosis 1 mg/kg | p.o. Dosis 3 mg/kg | p.o. Dosis 10 mg/kg | Kontrolle |
| 0 | 3.99 ± 0.10 | 3.94 ± 0.14 | 4.01 ± 0.15 | 4.08 ± 0.12 |
| 15 | 6.07 ± 0.28 | 5.71 ± 0.23 | 5.06 ± 0.20 | 6.65 ± 0.40 |
| 30 | 6.01 ± 0.22 | 5.53 ± 0.19 | 5.11 ± 0.10 | 6.63 ± 0.10 |
| 60 | 6.34 ± 0.14 | 5.47 ± 0.15 | 4.82 ± 0.09 | 7.18 ± 0.30 |
| 120 | 5.45 ± 0.13 | 5.20 ± 0.16 | 4.50 ± 0.11 | 5.50 ± 0.09 |
| 240 | 4.57 ± 0.07 | 4.83 ± 0.15 | 4.29 ± 0.22 | 4.90 ± 0.07 |

Aus den in Tabelle 1 angegebenen Werten errechnet sich ein Grenzwert von 0,27 mg/kg Ratte und aus den in Tabelle 2 angegebenen Werten ein Grenzwert von 1,29 mg/kg Ratte (unter Grenzwert der Wirksubstanz wird diejenige Menge pro kg Versuchstier verstanden, bei deren Verabreichung nach einer Stunde noch eine deutliche Wirkung festzustellen ist).

Neben der Blutglukoseregulation kann man die erfindungsgemäßen Oligosaccharide auch zur Hemmung der Speichel α-Amylase verwenden. Dieses Enzyme bewirkt die Verdauung der Stärke im Mund und der so gebildete Zucker fördert den Kariesbefall der Zähne. Die erfindungsgemäßen Verbindungen können daher zur Verhütung oder Verminderung der Ausbildung von Karies angewandt werden.

Weiterhin können sie als biochemische Reagenzien sowie als diagnostische Mittel verwendet werden.

Beispiel 1: 5,0 g Inhibitorgemisch mit den Komponenten W-46 A, W-46 B und W-46 C, gewonnen entsprechend den Angaben in EP-A2-0 173 948 (Beispiele 1 und 2), werden in 80 ml 2 N Trifluoressigsäure gelöst und während 10 Minuten unter Sauerstoffausschluß auf 100 °C erhitzt. Nach Ablauf dieser Zeit wird das Reaktionsgefäß unter Rühren rasch abgekühlt und die wäßrige Trifluoressigsäure im Vakuum bis zur Trockene abdestilliert.

Zur Gewinnung der verkürzten Inhibitoren wird der feste Destillationsrückstand in destilliertem Wasser gelöst und auf eine, mit dem Ionenaustauscher SP-Sephadex$^R$ C-25 gefüllte und auf pH 3 äquilibrierte Säule von 3 l Inhalt aufgetragen. Die Elution der Wirksubstanz erfolgt durch Anlegen eines ansteigenden 50 mM NaCl-Gradienten. Während die Kochsalzkonzentration mit einer Leitfähigkeit von 3.0 mS den Inhibitor W-46 H von der Säule löst, geschieht die Elution der Verbindung W-46 P bei einer Leitfähigkeit von 4 mS. Die getrennt gesammelten Eluate werden zur Entsalzung durch Ultrafiltration bei ≧ 35 bar konzentriert und anschließend gefriergetrocknet. Es resultieren 0.7 g des Inhibitors W-46 H und 1.6 g des Inhibitors W-46 P (jeweils als Hydrochloride).

Beispiel 2: 65 g Inhibitorgemisch, gewonnen nach EP-A2-0 173 948 (Beispiele 1, 2), werden in 6,5 l Wasser gelöst und der pH-Wert wird auf 7.5 eingestellt. Anschließend wird 1 g α-Amylase aus Bacillus subtilis 130 U/mg hinzugegeben und 18 Stunden bei 60° gerührt. Während dieser Zeit achtet man darauf, daß der pH-Wert konstant bleibt. Nach Ablauf der Reaktionszeit wird die resultierende Lösung bei Raumtemperatur über 4 l 20 Polystyrol-Adsorptionsharz ($^R$Diaion HP-20) filtriert. Während der sogenannte Durchlauf die unerwünschten Verunreinigungen, Neutralzucker und Salze enthält gewinnt man den Inhibitor W-46 H durch Waschen der Säule mit 20 l 10 %iger Isopropanollösung. Die Trocknung führt zum gewünschten Inhibitor. Sollte an dieser Stelle die Verbindung W-46 H nicht rein genug anfallen, so kann eine Weiterreinigung an Ionenaustauschern erfolgen, wie in Beispiel 1 beschrieben.

Beispiel 3: 10 g des Inhibitors W-46 H gewonnen nach Beispiel 1 werden in 200 ml Wasser gelöst und über 200 ml Anionenaustauscher IRA-68, OH-Form, welches in eine Glassäule gefüllt wird, filtriert. Anschließend wird mit 200 ml Wasser nachgewaschen. Die Eluate werden vereinigt. Sie enthalten den Inhibitor in Form der freien Base. Die Lösung wird nun in 4 gleiche Teile geteilt. Je ein Viertel wird mit Glukuronsäure bzw. mit Schwefelsäure bzw. mit Salzsäure auf pH 6 gestellt und gefriergetrocknet ebenso wie das 4. Viertel, welches nicht neutralisiert wird. Die letzte Lösung ergibt nach der Trocknung 2,3 g W-46 H (freie Base) mit 4 x $10^4$ AIE/mg und 1 x $10^4$ SIE/mg, die anderen Lösungen 2,5 g Glucuronsaures W-46 H, spezifische Aktivität 3,6 x $10^4$ AIE/mg und 9 x $10^3$ SIE/mg bzw. 2,4 g Schwefelsaures W-46 H, spezifische Aktivität 3,8 x $10^4$ AIE und 9.6 x $10^3$ SIE/mg bzw. 2,34 g Salzsaures W-46 H, spezifische Aktivität 3,9 x $10^4$ AIE/mg und 1 x $10^4$ SIE/mg.

Beispiel 4: 10 g α-Glukosidaseinhibitor W-46 P gemäß Beispiel 1 werden behandelt wie in Beispiel 3 mit W-46 H beschrieben. Es resultieren 2.4 g W-46 P freie Base mit
1 x $10^4$ AIE/mg und
3 x $10^4$ SIE/mg
2.6 g W-46 P-Glucuronat
2.3 g W-46 P-Sulfat
2.3 g W-46 P-Chlorid

**Patentansprüche**
**Patentansprüche für folgende Vertragsstaaten : BE, CH, DE, FR, GB, IT, LI, LU, NL, SE**

1. Oxiran – Pseudoologosaccharide der Formel I

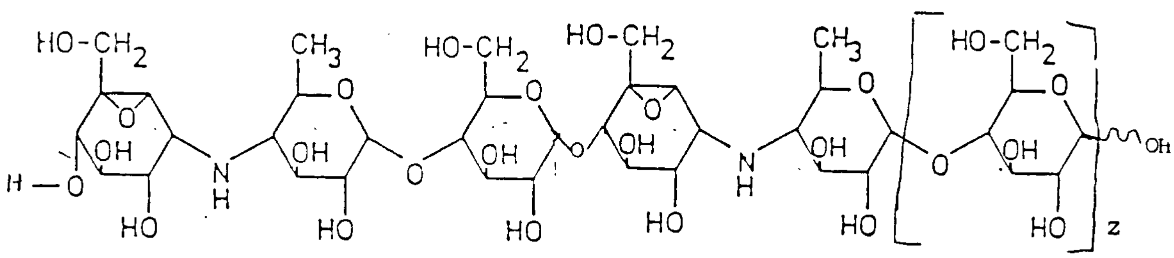

I

worin
z = null oder 1 ist,
sowie deren physiologisch verträgliche Salze mit Säuren.

2. Verfahren zur Herstellung der Oxiran – Pseudooligosaccharide gemäß Anspruch 1, dadurch gekenn – zeichnet, daß man
a) von einem α – Glukosidaseinhibitor der Formel II

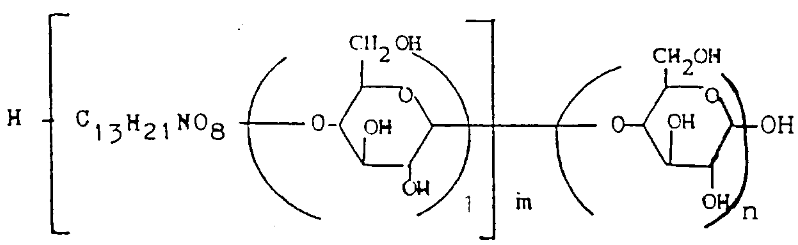

II

in welcher
m = 2 oder 3
n = eine ganze Zahl von 1 bis 20 bedeutet,
oder von einem Gemisch dieser Inhibitoren mit chemischen oder biochemischen Methoden unter Bildung einer Verbindung der Formel I Zucker abspaltet, oder
b) einen Pseudooligosaccharide der Formel I erzeugenden Streptomyceten in einem Fermenta – tionsmedium in geeignetem Submersverfahren kultiviert, die Pseudooligosaccharide aus dem Mycel oder dem Kulturfiltrat in an sich bekannter Weise isoliert und reinigt
und die erhaltenen Verbindungen der Formel I gegebenenfalls in ein physiologisch verträgliches Salz überführt.

3. Pharmazeutisches Präparat gekennzeichnet durch einen Gehalt an einem Oxiran – Pseudooligosac – charid gemäß Anspruch 1.

4. Verfahren zur Herstellung eines pharmazeutischen Präparates, dadurch gekennzeichnet, daß man ein Oxiran – Pseudooligosaccharid gemäß Anspruch 1 in eine geeignete Darreichungsform überführt.

5. Oxiran – Pseudooligosaccharide gemäß Anspruch 1 zur Anwendüng als Hemmstoffe der α – Glukosi – dase.

6. Oxiran – Pseudoologosaccharide gemäß Anspruch 1 zur Anwendüng als Arzneimittel gegen Diabetes, Prädiabetes und Adipositas.

7. Oxiran-Pseudooligosaccharide gemäß Anspruch 1 zur Anwendüng als Diagnostikum, Reagenz oder Prophylaktikum gegen Karies.

8. Oxiran-Pseudoologosaccharide gemäß Anspruch 1 zur Anwendüng als Arzneimittel.

**Patentansprüche für folgende Vertragsstaaten : GR, ES, AT**

1. Verfahren zur Herstellung von Oxiran-Pseudoologosacchariden der Formel I

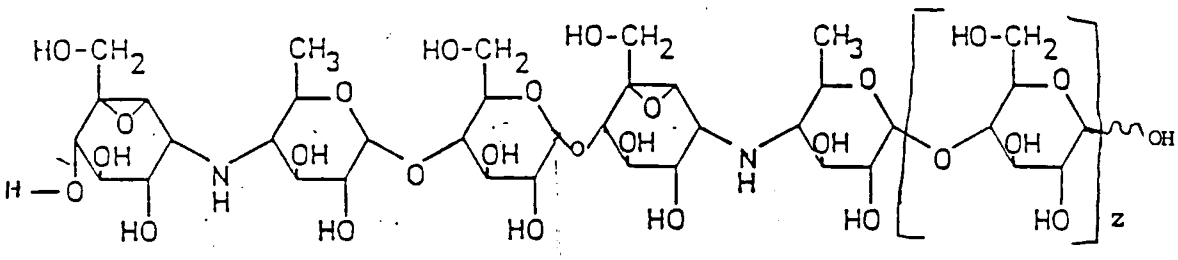

I

worin
z = null oder 1 ist,
sowie von deren physiologisch verträglichen Salzen mit Säuren, dadurch gekennzeichnet, daß man
a) von einem $\alpha$-Glukosidaseinhibitor der Formel II

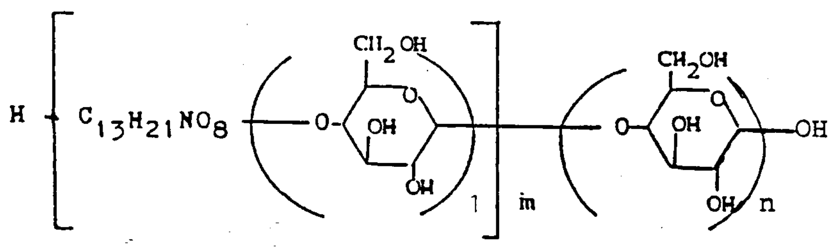

II

in welcher
m = 2 oder 3
n = eine ganze Zahl von 1 bis 20 bedeutet,
oder von einem Gemisch dieser Inhibitoren mit chemischen oder biochemischen Methoden unter Bildung einer Verbindung der Formel I Zucker abspaltet, oder
b) einen Pseudooligosaccharide der Formel I erzeugenden Streptomyceten in einem Fermentationsmedium in geeignetem Submersverfahren kultiviert, die Pseudooligosaccharide aus dem Mycel oder dem Kulturfiltrat in an sich bekannter Weise isoliert und reinigt
und die erhaltenen Verbindungen der Formel I gegebenenfalls in ein physiologisch verträgliches Salz überführt.

2. Verfahren zur Herstellung eines pharmazeutischen Präparates, dadurch gekennzeichnet, daß man ein Oxiran-Pseudooligosaccharid der in Anspruch 1 angegebenen Formel I oder ein physiologisch verträgliches Salz davon in eine geeignete Darreichungsform überführt.

3. Verfahren gemäß Anspruch 2, dadurch gekennzeichnet, daß das pharmazeutische Präparat zur Anwendüng als Diagnostikum, Reagenz oder Prophylaktikum gegen Karies dienen soll.

## Claims

### Claims for the following Contracting States : BE, CH, DE, FR, GB, IT, LI, LU, NL, SE

**1.** An oxirane-pseudooligosaccharide of the formula I

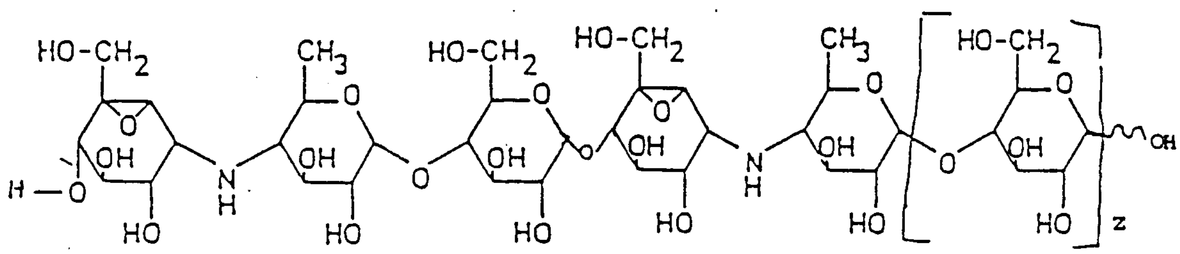

I

in which

z is zero or 1,

and its physiologically acceptable salts with acids.

**2.** A process for the preparation of an oxirane-pseudooligosaccharide as claimed in claim 1, which comprises

a) eliminating sugar from an $\alpha$-glucosidase inhibitor of the formula II

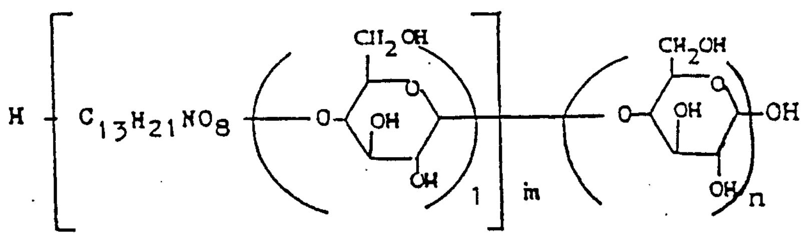

II

in which

m denotes 2 or 3, and

n denotes an integer from 1 to 20,

or from a mixture of these inhibitors using chemical or biochemical methods, forming a compound of the formula I,

or

b) cultivating, in a fermentation medium using a suitable submersion method, a Streptomycete which produces pseudooligosaccharides of the formula I, isolating and purifying the pseudooligosaccharides from the mycelium or culture filtrate in a fashion which is known per se, and converting, if appropriate, the compounds of the formula I obtained into a physiologically acceptable salt.

**3.** A pharmaceutical preparation containing an oxirane-pseudooligosaccharide as claimed in claim 1.

**4.** A process for the preparation of a pharmaceutical preparation,wherein an oxirane-pseudooligosaccharide as claimed in claim 1 is converted into a suitable form of administration.

**5.** An oxirane-pseudooligosaccharide as claimed in claim 1 for use as an inhibitor of $\alpha$-glucosidase.

**6.** An oxirane-pseudooligosaccharide as claimed in claim 1 for use as a medicament against diabetes, pre-diabetes and adiposis.

**7.** An oxirane-pseudooligosaccharide as claimed in claim 1 for use as a diagnostic agent, reagent or prophylactic against caries.

**8.** An oxirane-pseudooligosaccharide as claimed in claim 1 for use as a medicament.

**Claims for the following Contracting States : GR, ES, AT**

**1.** A process for the preparation of an oxirane-pseudooligosaccharide of the formula I

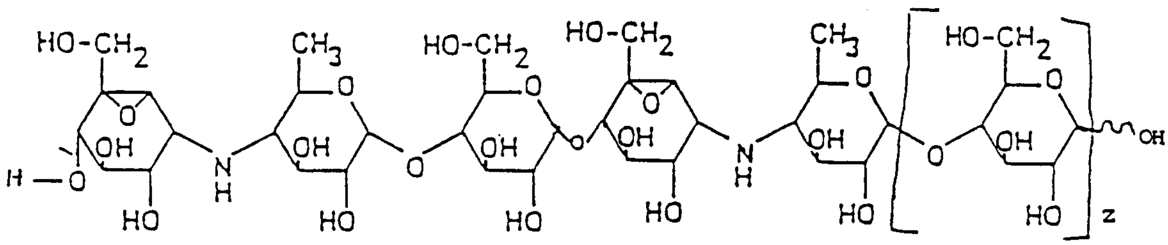

I

in which

z is zero or 1,

and its physiologically acceptable salts with acids, which comprises

a) eliminating sugar from an α-glucosidase inhibitor of the formula II

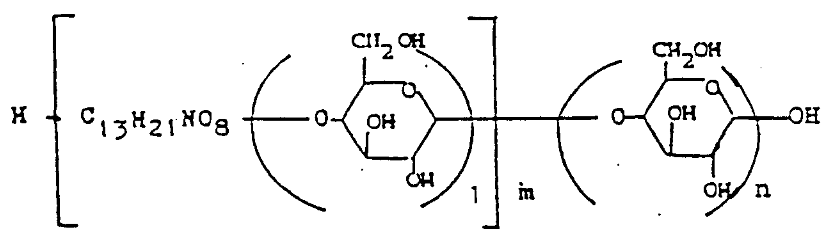

II

in which

m denotes 2 or 3, and

n denotes an integer from 1 to 20,

or from a mixture of these inhibitors using chemical or biochemical methods, forming a compound of the formula I,

or

b) cultivating, in a fermentation medium using a suitable submersion method, a Streptomycete which produces pseudooligosaccharides of the formula I, isolating and purifying the pseudooligosaccharides from the mycelium or culture filtrate in a fashion which is known per se, and converting, if appropriate, the compounds of the formula I obtained into a physiologically acceptable salt.

**2.** A process for the preparation of a pharmaceutical preparation, wherein an oxirane-pseudooligosaccharide of the formula I specified in claim 1, or a physiologically acceptable salt thereof, is converted into a suitable form of administration.

**3.** The process as claimed in claim 2, wherein the pharmaceutical preparation is intended to be used as a diagnostic agent, reagent or prophylactic against caries.

**Revendications**

**Revendications pour les Etats contractants suivants : BE, CH, DE, FR, GB, IT, LI, LU, NL, SE**

1. Oxirane – pseudo – oligosaccharides de formule I

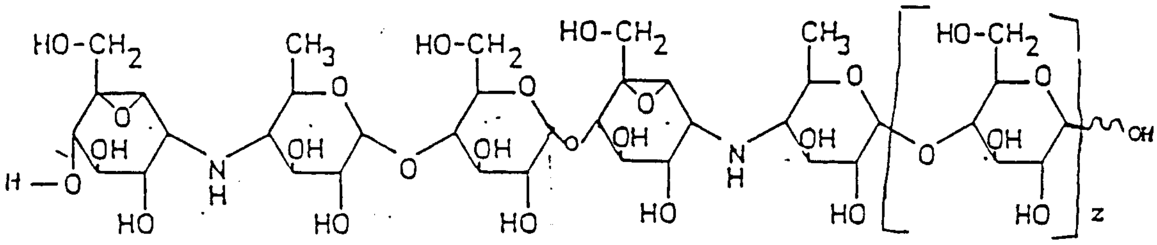

I

où

z = 0 ou 1,

ainsi que les sels physiologiquement acceptables de ceux – ci formés avec des acides.

2. Procédé pour préparer les oxirane – pseudo – oligosaccharides selon la revendication 1, caractérisé en ce que:

a) on élimine le sucre d'un inhibiteur de l'$\alpha$ – glucosidase de formule II

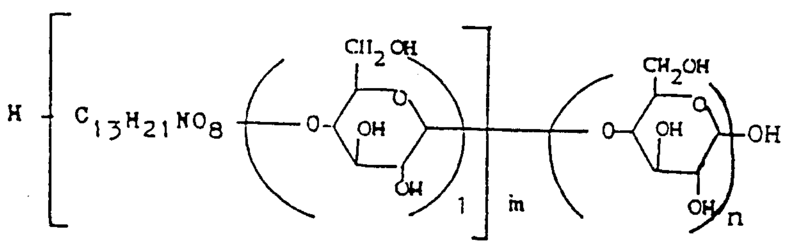

II

dans laquelle

m = 2 ou 3

n = un nombre entier entre 1 et 20,

ou d'un mélange d'inhibiteurs de ce type, au moyen de méthodes chimiques ou biochimiques, ce qui forme un composé de formule I, ou

b) on cultive un streptomycète produisant des pseudo – oligosaccharides de formule I dans un milieu de fermentation, à l'aide d'un procédé par submersion convenable, on isole les pseudo – oligosaccharides à partir du mycélium ou du filtrat de culture, d'une manière connue par elle – même, et on les purifie

et, le cas échéant, on transforme les composés de formule I obtenus en un sel physiologiquement acceptable.

3. Préparation pharmaceutique caractérisée en ce qu'elle contient de l'oxirane – pseudo – oligosaccharide selon la revendication 1.

4. Procédé pour fabriquer une préparation pharmaceutique, caractérisé en ce qu'on met l'oxirane – pseudo – oligosaccharide selon la revendication 1 sous une forme de présentation appropriée.

5. Oxirane – pseudo – oligosaccharides selon la revendication 1 à utiliser comme inhibiteurs de l'$\alpha$ – glucosidase.

6. Oxirane – pseudo – oligosaccharides selon la revendication 1 à utiliser comme médicaments contre le diabète, le prédiabète et l'obésité.

7. Oxirane-pseudo-oligosaccharides selon la revendication 1 à utiliser comme agents de diagnostic, réactifs ou agents prophylactiques contre les caries.

8. Oxirane-pseudo-oligosaccharides selon la revendication 1 à utiliser comme médicaments.

**Revendications pour les Etats contractants suivants : GR, ES, AT**

1. Procédé pour préparer l'oxirane-pseudo-oligosaccharides de formule I

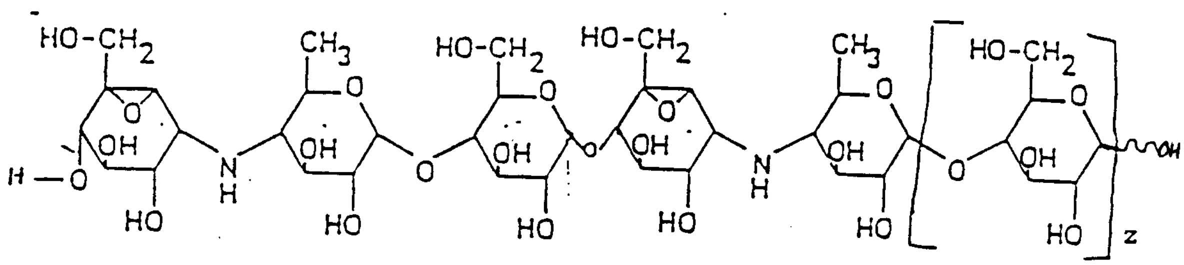

I

où
z = 0 ou 1,
ainsi que des sels physiologiquement acceptables de ceux-ci, formés avec des acides, caractérisé en ce que:
a) on élimine le sucre d'un inhibiteur de l'α-glucosidase de formule II

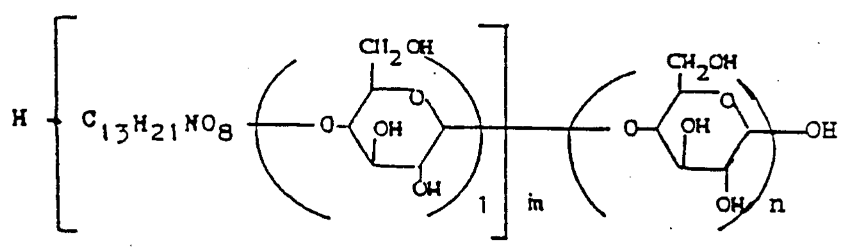

II

dans laquelle
m = 2 ou 3
n = un nombre entier entre 1 et 20,
ou d'un mélange d'inhibiteurs de ce type, au moyen de méthodes chimiques ou biochimiques, ce qui forme un composé de formule I, ou
b) on cultive un streptomycète produisant des pseudo-oligosaccharides de formule I dans un milieu de fermentation, à l'aide d'un procédé par submersion convenable, on isole les pseudo-oligosaccharides à partir du mycélium ou du filtrat de culture, d'une manière connue par elle-même, et on les purifie
et, le cas échéant, on transforme les composés de formule I obtenus en un sel physiologiquement acceptable.

2. Procédé pour fabriquer une préparation pharmaceutique, caractérisé en ce qu'on met l'oxirane-pseudo-oligosaccharide de formule I, citée dans la revendication 1, ou un sel physiologiquement acceptable de celui-ci, sous une forme de présentation appropriée.

3. Procédé selon la revendication 2, caractérisé en ce que la préparation pharmaceutique est utilisable comme agent de diagnostic, réactif ou agent prophylactique contre les caries.